# EUROPEAN PATENT SPECIFICATION

(11) **EP 1 824 349 B1**
(45) Date of publication and mention of the grant of the patent: **13.02.2013**
(21) Application number: 05814324.9
(22) Date of filing: 12.12.2005
(51) Int. Cl.: A23L 1/076, A23L 1/30

(54) **PRODUCTION PROCESS FOR MAKING DIETETIC PRODUCT - ORAL TABLETS CONTAINING BEEBREAD AND VITAMIN C**
PRODUKTIONSVERFAHREN ZUR HERSTELLUNG EINES DIÄTPRODUKTS - ORALTABLETTEN, DIE BIENENBROT UND VITAMIN C ENTHALTEN
PROCÉDÉ DE FABRICATION D'UN PRODUIT DIÉTÉTIQUE - TABLETTES CONTENANT DU PAIN D'ABEILLE ET DE LA VITAMINE C

(30) Priority: 13.12.2004 YU 108104
(43) Date of publication of application: 29.08.2007
(73) Proprietor: Mogorovic, Milos, 11000 Beograd (YU)
(72) Inventor: Mogorovic, Milos, 11000 Beograd (YU)
(74) Representative: Hofstetter, Schurack & Partner
(86) International application number: PCT/YU2005/000030
(87) International publication number: WO 2006/066283

(56) References cited:
- US-A- 4 426 397
- DATABASE WPI Section Ch, Week 200127 Derwent Publications Ltd., London, GB; Class B04, AN 2001-264619 XP002369155 & RU 2 161 977 C1 (SIBGATULLIN ZH ZH) 20 January 2001 (2001-01-20)
- DATABASE WPI Section Ch, Week 199815 Derwent Publications Ltd., London, GB; Class B05, AN 1998-167664 XP002369156 & RU 2 086 245 C1 (SIBGATULLIN ZH ZH) 10 August 1997 (1997-08-10)
- PATENT ABSTRACTS OF JAPAN vol. 017, no. 315 (C-1071), 16 June 1993 (1993-06-16) & JP 05 030926 A (RYOJU SHOJI KK), 9 February 1993 (1993-02-09)
- NAGAI T ET AL: "Preparation and functional properties of extracts from bee bread." NAHRUNG/FOOD, vol. 3, no. 48, 2004, pages 226-229, XP002369152

## Description

### FIELD OF ART TO WHICH THIS INVENTION APPLIES

The subject of this invention, generally speaking, falls into the category of dietetic refreshing products in the form of tablets and specifically relates to oral tablets containing beebread and vitamin C and its production process.

### STATE OF THE ART

In the state of the art there are several technical solutions of dietetic products with concentrated vitamins and other refreshing and nutrient materials.

One of the prior art documents in this technical field is, for example, the RU 2 161 977 C1. The process disclosed therein comprises the steps of drying beebread up to a humidity index of 15%, followed by processing with technological air. To extend its shelf life, the resulting product is disinfected, cooled down and extracted in granular form. Subsequently, the granulae are dried to a humidity index not exceeding 1%. The drying is accomplished by heating the granulae under a pressure of 100 mmHG to a temperature of 45°C in a vacuum chamber. The granulae are simultaneously mixed with several components and finally ground together with ascorbic acid to obtain a homogenous powder.

From the US 4 426 397 A1 it can be gathered to be known to mix, evaporate, dry, and finally grind bee products such as pollen, propolis, beebread, honey or wax with milk product concentrates at temperatures of up to 75°C, in order to extend their shelf lives. In this process it is envisaged that 25% to 70% of the dry weight stem from the milk products. Furthermore, various artificial additives and preservatives are added to the mixture in order to extend its shelf life.

The RU 2 086 245 C 1 discloses a method for obtaining a product from beebread in which it is dried in a vacuum chamber by means of a pressure of 100 mmHG through irradiation with microwaves to a humidity not exceeding 1%.

From the JP 05 030926 A a method may be gathered in which an inexpensive, powdered dietetic supplement with good digestibility properties is produced by way of fermenting, drying and powdering isolated and purified pollen instead of beebread. Various vitamins are added to the dietetic supplement.

### TECHNICAL PROBLEM

The technical problem resolved with this invention consists of the following: how to produce dietetic oral tablets with precisely prescribed doses of beebread that have a strengthening effect on the immune system and have a much longer shell life than beebread in its natural form, contain no artificial preservatives and coloring and allow their use in various conditions.

### FUNDAMENTAL NATURE OF INVENTION

The fundamental nature of this invention is the fact that the following raw materials are used for production of dietetic oral tablets: ether orange oil, SiO₂ (micronized silicium dioxide), malt dextrin, beta carotene, sodium riboflavin phosphate, sucrose, mannitol, lactose of which, before all, are those basic ones, lyophilized beebread and vitamin C, natural with tested positive effects that in a well balanced ratio have, apart from refreshing influence, a stimulating effect on the human body.

The fundamental nature of the invention is the fact that according to the mentioned process, by using lyophilized beebread, duration of its qualitative physiological effects is considerably prolonged. Such use of beebread prevents use of preserves and additives and this improves product quality from the health point of view.

One innovation is the fact that biologically useful substances from beebread can be absorbed through the mouth mucous membrane so that their decomposition is not performed in the gastrointestinal tract.

With regard to the well-known dietetic oral tablets known to the author, the subject invention has several advantages over them like the following:
- it can be used by persons of all ages, children, adults, pregnant and nursing women;
- its preventive properties are undisputable as there is an evident immunological effect;
- it does not increase body weight;
- it is produced relatively easily and simply and the process for its production does not require special devices and costly technological equipment.

### DETAILED DESCRIPTION OF INVENTION

First raw materials to produce dietetic oral tablets containing beebread and vitamin C are:
- lyophilized beebread
- vitamin C
- ether orange oil
- SiO₂ (micronized silicium dioxide)
- malt dextrin
- beta carotene
- sodium riboflavin phosphate
- sucrose
- lactose
- mannitol

*Beebread* (thanks to its healing properties has been widely used in medicine since ancient times), which is the most important ingredient of oral tablets according to the invention, has a high biological value and contains proteins, vitamins and oligo-elements in superbly balanced quantities that are physiologically acceptable to human body.

*Vitamin C* (has a protective and bio-stimulating effect) is part of enzyme systems of our body and is necessary for its normal functioning, maintenance and improvement of the immune system.

*Ether orange oil* gives adequate aroma.

*SiO*₂ prevents agglomeration of particles and allows easier redispersion and flow. By using SiO₂ ether oil is converted from the liquid to the solid state and such state remains unchanged. It is also possible to use MgO instead of SiO₂ and this is a matter of choice by the manufacturer.

*Malt dextrin* is also used as a carrier for converting ether orange oil to the solid state and for adjustment of optimum concentration in the process of pre-preparation of powdered aroma for the production of oral tablets..

*Beta carotene* is a provitamin A that in this example of the invention is used as color. It is in the liquid state as a raw material and the same SiO₂ and malt dextrin effect applies to it.

*Sodium riboflavin phosphate* is in the solid state and serves to attain the desired color of oral tablets.

*Sucrose, lactose and mannitol* are used as carriers.

For easier production or due to lack of some raw materials on the market it is possible to use natural aroma or ready natural powdered aroma and color for making the subject oral tablets and on the market they can be found under different commercial names and are manufactured by companies FRUKTAMIN, ZIVODAN RUR, ETOL, etc. in various concentrations.

The process for the production of the subject dietetic products is carried out in the following stages:
First stage in the production of the subject dietetic product is to convert aroma and color to the solid state. Firstly, ether orange oil 6,667 mass % and beta carotene 0,134 mass % is coarsely mixed with SiO₂ 13,334-26,667 mass % after which sodium riboflavin phosphate is added 0,267 mass % and supplemented with malt dextrin up to 100 mass %, it is all mixed and sieved through a chromium metal or plastic sieve with openings of 0,75 mm. Coarse mixing of the mentioned components is done during the first stage manually and then in a standard chromium granulator. It is necessary to mention that at this stage during the conversion of aroma and color to the solid state, larger quantities of raw materials are used for precise dosing.

The next stage consists of preparation of beebread triturate 1: 10 so that 10 mass % of lyophilized beebread is coarsely mixed with 90 mass % of sucrose in a homogenous mixture that is sieved through a sieve with openings of 0,75 mm. At this stage larger quantities of raw materials are used for precise dosing.

Sucrose is firstly prepared so that it is ground in a standard chromium hammering mill to the granulate of specific fineness and 40% of granulate passes through the sieve with openings of 0,75 mm and the rest through the sieve of openings of 1 mm.

Vitamin C is prepared so that it is sieved through a sieve with openings of 0,75 mm.

Mannitol and lactose are sieved through a sieve with openings of 0,75 mm.

All substances taking part in the production of the subject oral tablets and prepared according to the above-mentioned stages are measured precisely in the following mass %:
- beebread triturate 7,2 mass %
- vitamin C 5,8 mass %
- mixture of aroma and color 6,76 mass %
- sucrose 22 mass %
- lactose 29 mass %
- mannitol 29 mas %
- SiO₂ 0,24 mass%
and in the mentioned order are thrown in the chromium mixer and are mixed until a homogenous mixture is obtained. Duration of mixing varies and depends on mixer characteristics. If the mixer is a cube, double cone or the like, mixing lasts maximally 20 minutes. If the mixer has cutting blades and a knife or if it performs against-the-flow double mixing then mixing lasts maximally 5 minutes. Upon completed mixing, concentration of substance samples taken from various parts of the mixer can maximally differ up to:
- beebread 10%
- vitamin C 10%
- mixture of aroma and color 10%
- sucrose 5%
- lactose 5%
- mannitol 5%
- SiO₂ 10%

The granulate so obtained is then shaped in tablets on a standard machine for making tablets in the well-known manner and in the example of invention feasibility tool used for making tablets have had a diameter of 14 mm and thus tablets of 0,83 g have been produced with a maximum variation up to 5%.

Such tablets are then packed in bottles, hard or soft blister.

Ready products are kept at room temperature without presence of moisture or light.

In order to prove invention feasibility an example is given of production of oral tablets of the following raw materials: ether orange oil, SiO₂ (micronized silicium dioxide), malt dextrin, beta carotene, sodium riboflavin phosphate. Also, without reducing quality of oral tablets, ready natural dry orange aroma can be used mixed with natural colors or already prepared dry natural orange aroma with mixed natural colors.

Apart from the abovementioned, it is necessary to mention that the premises in which production of the subject invention is carried out (in addition to general conditions for the production of dietetic substances intended for human use) must fulfill conditions in respect of relative air humidity that must be adjusted according to outside temperature and be below 40% of relative humidity.

### METHOD OF INDUSTRIAL OR OTHER APPLICATION OF INVENTION

The subject invention, according to this example, can be produced in plants having registration, adequate equipment and staff for the manufacturing of dietetic products.

Subject production is easy to organize based on description given in this invention application.

## Claims

1. The production process for making dietetic oral tablets IS **CHARACTERIZED BY** THAT the following raw materials are used and measured in the following mass proportions: lyophilized beebread 0,72 mass%, vitamin C 5,8 mass%, ether orange oil 0,45 mass%, SiO₂ 1,14-2,04 mass%, malt dextrin 5,363 - 4,463 mass%, beta carotene 0,009 mass%, sodium riboflavin phosphate 0,018 mass%, sucrose 28,5 and lactose 29 mass%.

2. Process for making dietetic oral tablets according to claim 1 IS **CHARACTERIZED BY** THAT the substances taking part in the production of oral tablets in precisely measured mass percentages: beebread triturate 7,2 mass% sieved through a sieve with openings 0,75 mm, vitamin C 5,8 mass% sieved through a sieve with openings 0,75 mm, mixture of aroma and color 6,76 mass% sieved through a sieve with openings of 0,75 mm, sucrose 22 mass% sieved through a sieve with openings of 1 mm, lactose 29 mass%, mannitol 29 mass% sieved through a sieve with openings of 0,75 mm and SiO₂ 0,24 mass% according to the mentioned order are thrown in the chromium mixer and are mixed until a homogenous mixture is obtained and after this granulate so obtained is shaped into tablets on a standard machined for making tablets weighing 0,83 g and then bottled, hard or soft blister.

3. Process for making dietetic oral tablets according to claim 1 or 2 IS **CHARACTERIZED BY** THAT aroma and color are converted to the solid state so that ether orange oil and beta carotene are coarsely mixed with SiO₂ after which sodium riboflavin phosphate is added and supplemented with malt dextrin and then it is all mixed and sieved through a sieve with openings of 0,75 mm.

4. Process for making dietetic oral tablets according to one of claims 1 to 3 IS **CHARACTERIZED BY** THAT beebread and vitamin C are coarsely mixed with sucrose in a homogenous mixture and then sieved through a sieve with openings of 0,75 mm.

5. Dietetic oral tablet containing beebread and vitamin C **CHARACTERIZED BY** THAT it is obtainable by a process according to one of claims 1 to 4.

## Patentansprüche

1. Produktionsverfahren zur Herstellung von diätetischen Tabletten zum Einnehmen, **dadurch gekennzeichnet, dass** die folgenden Rohmaterialien verwendet werden und in den folgenden Massenverhältnissen dosiert werden: lyophilisiertes Bienenbrot 0,72 Massen-%, Vitamin C 5,8 Massen-%, Etherorangenöl 0,45 Massen-%, SiO₂ 1,14-2,04 Massen-%, Malzdextrin 5,363 - 4,463 Massen-%, Betacarotin 0,009 Massen-%, Natriumriboflavinphosphat 0,018 Massen-%, Saccharose 28,5 und Lactose 29 Massen-%.

2. Verfahren zur Herstellung von diätetischen Tabletten zum Einnehmen nach Anspruch 1, **dadurch gekennzeichnet, dass** die Substanzen, die an der Herstellung von Tabletten zum Einnehmen beteiligt sind, in genau dosierten Massenprozentsätzen: Bienenbrottriturat 7,2 Massen-%, gesiebt durch ein Sieb mit Öffnungen von 0,75 mm, Vitamin C 5,8 Massen%, gesiebt durch ein Sieb mit Öffnungen von 0,75 mm, ein Gemisch von Aroma und Farbe 6,76 Massen-%, gesiebt durch ein Sieb mit Öffnungen von 0,75 mm, Saccharose 22 Massen-%, gesiebt durch ein Sieb mit Öffnungen von 1 mm, Lactose 29 Massen-%, Mannit 29 Massen-%, gesiebt durch ein Sieb mit Öffnungen von 0,75 mm, und SiO₂ 0,24 Massen-% gemäß der erwähnten Reihenfolge in den Chrommischer geschüttet werden und vermischt werden, bis ein homogenes Gemisch erhalten wird, und danach dieses so erhaltene Granulat an einer Standardmaschine zur Herstellung von Tabletten zu Tabletten geformt wird, die 0,83 g wiegen, die dann in Flaschen gefüllt, in harten oder weichen Blisterverpackungen verpackt werden.

3. Verfahren zur Herstellung von diätetischen Tabletten zum Einnehmen nach Anspruch 1 oder 2, **dadurch gekennzeichnet, dass** Aroma und Farbe in den festen Zustand umgesetzt werden, so dass Etherorangenöl und Betacarotin mit SiO₂ grob vermischt werden, wonach Natriumriboflavinphosphat zugegeben und mit Malzdextrin ergänzt wird und dann alles vermischt und durch ein Sieb mit Öffnungen von 0,75 mm gesiebt wird.

4. Verfahren zur Herstellung von diätetischen Tabletten zum Einnehmen nach einem der Ansprüche 1 bis 3, **dadurch gekennzeichnet, dass** Bienenbrot und Vitamin C mit Saccharose in einem homogenen Gemisch grob vermischt werden und dann durch ein Sieb mit Öffnungen von 0,75 mm gesiebt werden.

5. Diätetische Tablette zum Einnehmen, die Bienenbrot und Vitamin C enthält, **dadurch gekennzeichnet, dass** sie durch ein Verfahren nach einem der Ansprüche 1 bis 4 erhältlich ist.

## Revendications

1. Processus de production, destiné à fabriquer des comprimés oraux diététiques, **caractérisé en ce que** les matières premières suivantes sont utilisées et mesurées dans les proportions en masse suivantes : 0,72 % en masse de pain d'abeille lyophilisé, 5,8 % en masse de vitamine C, 0,45 % en masse d'huile d'éther orange, 1,14 - 2,04 % en masse de SiO₂, 5,363 - 4,463 % en masse de dextrine de malt, 0,009 % en masse de bêta-carotène, 0,018 % en masse de phosphate sodique de riboflavine, 28,5 % en masse de saccharose et 29 % en masse de lactose.

2. Processus, destiné à fabriquer des comprimés oraux diététiques selon la revendication 1, **caractérisé en ce que** les substances, qui prennent part à la production de comprimés oraux selon des pourcentages en masse, mesurés avec précision : 7,2 % en masse de triturat de pain d'abeille, passés à travers un tamis, doté d'ouvertures de 0,75 mm, 5,8 % en masse de vitamine C, passés à travers un tamis, doté d'ouvertures de 0,75 mm, 6,76 % en masse d'un mélange d'arôme et de colorant, passés à travers un tamis, doté d'ouvertures de 0,75 mm, 22 % en masse de saccharose, passés à travers un tamis, doté d'ouvertures de 1 mm, 29 % en masse de lactose, 29 % en masse de mannitol, passés à travers un tamis, doté d'ouvertures de 0,75 mm et 0,24 % en masse de SiO₂, suivant l'ordre mentionné, sont projetées dans le mélangeur, revêtu de chrome et sont mélangées, jusqu'à ce qu'un mélange homogène soit obtenu et ce granulé, ainsi obtenu, est formé, par la suite, pour obtenir des comprimés, sur une machine normalisée, destinée à fabriquer des comprimés qui pèsent 0,83 g puis embouteillés, mis sous emballage thermoformé dur ou souple.

3. Processus, destiné à fabriquer des comprimés oraux diététiques selon la revendication 1 ou 2, **caractérisé en ce qu'**arôme et colorant sont passés à l'état solide, de sorte que l'huile essentielle d'orange et le bêta-carotène sont mélangés grossièrement avec du SiO₂, après quoi du phosphate sodique de riboflavine est ajouté et complété par de la dextrine de malt puis le tout est mélangé et passé à travers un tamis, doté d'ouvertures de 0,75 mm.

4. Processus, destiné à fabriquer des comprimés oraux diététiques selon l'une des revendications 1 à 3, **caractérisé en ce que** du pain d'abeille et de la vitamine C sont mélangés grossièrement avec du saccharose, pour obtenir un mélange homogène puis passés à travers un tamis, doté d'ouvertures de 0,75 mm.

5. Comprimé oral diététique, contenant du pain d'abeille et de la vitamine C, **caractérisé en ce qu'**il peut être obtenu par un processus selon l'une des revendications 1 à 4.
